(19) Europäisches Patentamt
European Patent Office
Office européen des brevets

(11) Publication number: 0 214 862 B1

(12) # EUROPEAN PATENT SPECIFICATION

(45) Date of publication of patent specification: **13.03.91 Bulletin 91/11**

(51) Int. Cl.$^5$: **C07C 15/00**, A61K 31/28

(21) Application number: **86306914.2**

(22) Date of filing: **08.09.86**

(54) **Anti-tumour platinum complexes, their preparation and their use.**

The file contains technical information submitted after the application was filed and not included in this specification

(30) Priority: **06.09.85 JP 197097/85**

(43) Date of publication of application: **18.03.87 Bulletin 87/12**

(45) Publication of the grant of the patent: **13.03.91 Bulletin 91/11**

(84) Designated Contracting States: **AT BE CH DE FR GB IT LI LU NL SE**

(56) References cited:
**FR-A- 2 283 692**
**US-A- 4 419 351**

(73) Proprietor: **SANKYO COMPANY LIMITED**
**No. 1-6, 3-chome Nihonbashi Honcho Chuo-ku**
**Tokyo (JP)**

(72) Inventor: **Sugimura, Yukio, c/o Sankyo Chemical Res.Labs.**
**Sankyo Co. Limited, 2-58, 1-chome, Hiromachi Shinagawa-ku, Tokyo 140 (JP)**
Inventor: **Kameyama, Yukiko, c/o Sankyo Chemical Res.Labs.**
**Sankyo Co. Limited, 2-58, 1-chome, Hiromachi Shinagawa-ku, Tokyo 140 (JP)**
Inventor: **Hashimoto, Toshihiko, c/o Sankyo Chemical Res.Labs**
**Sankyo Co. Limited, 2-58, 1-chome, Hiromachi Shinagawa-ku, Tokyo 140 (JP)**
Inventor: **Shimizu, Fusaaki, c/o Sankyo Biological Res.Labs.**
**Sankyo Co. Limited, 2-58, 1-chome, Hiromachi Shinagawa-ku, Tokyo 140 (JP)**
Inventor: **Kitamura, Kouichi, c/o Sankyo Biological Res.Labs.**
**Sankyo Co. Limited, 2-58, 1-chome, Hiromachi Shinagawa-ku, Tokyo 140 (JP)**

(74) Representative: **Gibson, Christian John Robert et al**
**MARKS & CLERK 57/60 Lincoln's Inn Fields London WC2A 3LS (GB)**

EP 0 214 862 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a series of novel complexes of quadricoordinate, divalent platinum with a cyclic urea derivative. Such complexes have valuable anti-tumour activity with better solubility than known anti-tumour platinum compounds and complexes. The invention also provides a process for preparing these complexes and methods and compositions using them.

Cancerous disorders are a major medical problem but are often difficult to cure. The main reason for this is that the differences between tumour cells and normal cells are generally extremely small with the result that compounds which are toxic to tumour cells are also toxic to normal cells. The chemotherapy of cancerous disorders is generally dependent upon very limited differences between the susceptibilities of tumour cells and normal cells to anti-tumour agents.

Various platinum compounds are known to have anti-tumour activities. For example cisplatin [Rosenberg et al, Nature 222, 385 (1965) and The Merck Index, tenth edition (1983) monograph 2289] has been successfully used in the treatment of tumours and malonato(1,2-diaminocyclohexane)platinum(11) [e.g. US Patent No. 4,169,846 and Kidani et al, Gann, 71, 637-643 (1980)] has been proposed for such use. Both of the above platinum complexes have some structural similarity to the compounds of the invention. Another platinum complex, although structurally less similar to the compounds of the invention, which has recently become available for the treatment of tumours is carboplatin [Drugs of the Future, Vol. 8, No. 6, p489-491 (1983)]. However, most of the known platinum complexes, including those referred to above, have a high renal toxicity and a poor solubility in water, which makes it difficult to formulate them into an appropriate dosage form.

We have now discovered a novel series of platinum complexes which have good anti-tumour activity but relatively few side effects, such as renal toxicity and bone marrow suppression, and which have a good solubility in water.

The compounds of the present invention are quadricoordinate, divalent platinum complexes with cyclic urea derivatives, said complexes being represented by the formula (I) :

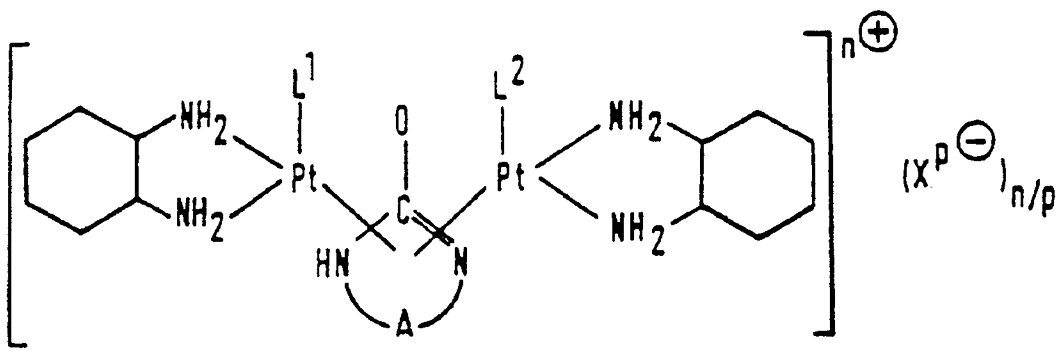

(I)

wherein

A represents an alkylene group having 2 or 3 carbon atoms ;

$L^1$ and $L^2$ are as specified in detail hereinafter in general, $L^1$ and $L^2$ are the same or different and each represents a neutral ligand or a monovalent or divalent anion, or $L^1$ and $L^2$ are linked together to form a monovalent anion, a divalent anion or a group of formula (II) :

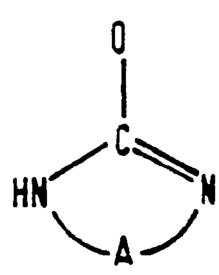

(II)

where A is as defined above :

$X^{p-}$ represents an anion of valency p being a nitrat ion, perchlorate ion, sulphate ion or car carbonate ion and

n is 0, 1 or 2.

The compounds of the invention may be prepared by reacting a platinum complex of formula (III) :

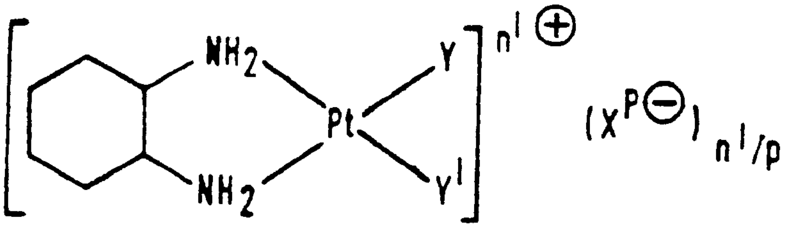
(III)

in which

$X^{p-}$ is as defined above ;

n′ is 0, 1 or 2 ; and

Y and Y′ are the same or different and each represents water, a nitrate ion, a hydroxide ion or a perchlorate ion or Y and Y′ are linked together to represent a sulphate or carbonate ion or a group of formula (IV) :

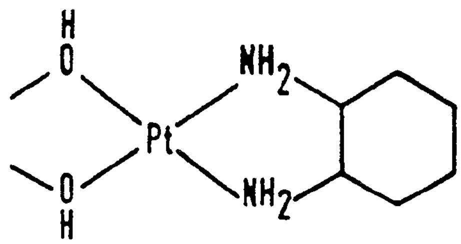
(IV)

with a compound of formula (V) :

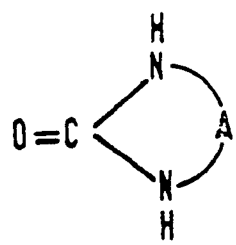
(V)

(in which A is as defined above).

The invention still further provides a composition comprising an anti-tumour agent and a pharmaceutically acceptable carrier or diluent, wherein the anti-tumour agent is at least one compound of formula (I).

The invention still further provides the use for the manufacture of a medicament for the treatment of an animal, preferably a mammal (including human beings) suffering from a tumour of a compound of formula (I).

The compounds of the present invention have at least one ligand which is a cyclic urea derivative of formula (II) and may, depending upon the meanings ascribed to $L^1$ and $L^2$ have two such ligands. In this ligand or these ligands, it is believed that the attachment to one of the platinum atoms is through the nitrogen atom of the –NH– group and the attachment to the other platinum atom is through the oxygen atom of the group.

A represents an alkylene group having 2 or 3 carbon atoms, which group may be a straight or branched chain group. The two "free" valencies of the alkylene group may be, and preferably are, attached to different carbon atoms, or they may be attached to the same carbon atom (in which case the group is sometimes referred to as an "alkylidene" group).

Examples of such groups include :

$-(CH_2)_2-$ : $CH_3-CH <$ : $-CH(CH_3)CH_2-$ : or $-(CH_2)_3-$

Of these, we prefer the groups of formula

$-(CH_2)_2-$ and $-(CH_2)_3-$.

Where $L^1$ or $L^2$ represents a neutral ligand, this is a water molecule, $H_2O$. Where $L^1$ or $L^2$ each represents

a monovalent or divalent anion.The resulting compound is pharmaceutically acceptable, that is to say it does not have reduced activity (or unacceptably reduced activity) or increased toxicity (or unacceptably increased toxicity) as compared to the parent compound with water as a ligand. To this end $L^1$ and/or $L^2$ is an inorganic anion which is the nitrate, halide (e.g. chloride, bromide or iodide), perchlorate, sulphate, carbonate or hydroxide anion or an organic anion which is the methoxide or ethoxide anions. We particularly prefer that $L^1$ and $L^2$, which may be the same or different, should each represent a nitrate, chloride, hydroxide, methoxide or ethoxide anion or $H_2O$. Alternatively, $L^1$ and $L^2$ may together represent a single one of the aforementioned anions. Alternatively, $L^1$ and $L^2$ together represent the nitrate, chloride, sulphate, hydroxide, methoxide, or ethoxide anions. Also preferred are the compounds where $L^1$ and $L^2$ together represent the aforementioned group of formula (II).

Where the compound of the invention includes an anion $X^{p-}$, it is pharmaceutically acceptable and is the nitrate, perchlorate, sulphate or carbonate anion, preferably the nitrate or sulphate anion.

The value represented by n is wholly dependent upon the nature of the ligands $L^1$ and $L^2$, since four positive charges are provided by the two divalent platinum atoms and a single negative charge is provided by the group of formula (II) shown in the formula (I), thus leaving a total of three positive charges to be balanced by $L^1$, $L^2$ and $X^{p-}$. For example, when $L^1$ and $L^2$ both represent monovalent anions, n is 1 ; when $L^1$ represents a neutral ligand and $L^2$ represents a monovalent anion, n is 2 ; and when one of $L^1$ and $L^2$ represents a divalent anion and the other represents a monovalent anion, n is 0.

It will be appreciated that a variety of isomers of the compounds of the invention are possible, depending upon the configuration of the two cyclohexanediamine molecules within the complex. For example, each cyclohexanediamine ligand may be the cis isomer, the trans isomer or a mixture of these and the two cyclohexanediamine ligands in the molecule may be the same isomer or different isomers. In the case of the trans isomers, optical activity is possible and these may be the d-enantiomer, the ℓ-enantiomer or a racemate.

The compounds of the invention may be synthesized by reacting a compound of formula (III), that is to say a compound of formula (IIIa) :

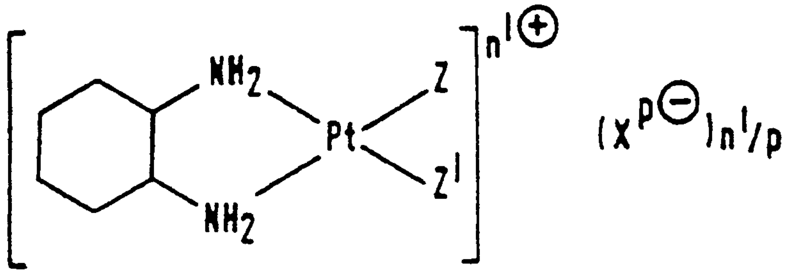

(IIIa)

(in which n′ and $X^{p-}$ are as defined above and Z and Z′ are the same or different and each represents a neutral ligand (for example water) or an anion (for example a nitrate, hydroxide or perchlorate anion) or Z and Z′ are linked together to represent an anion (preferably a sulphate or carbonate anion)], or a compound of formula (IIIb) :

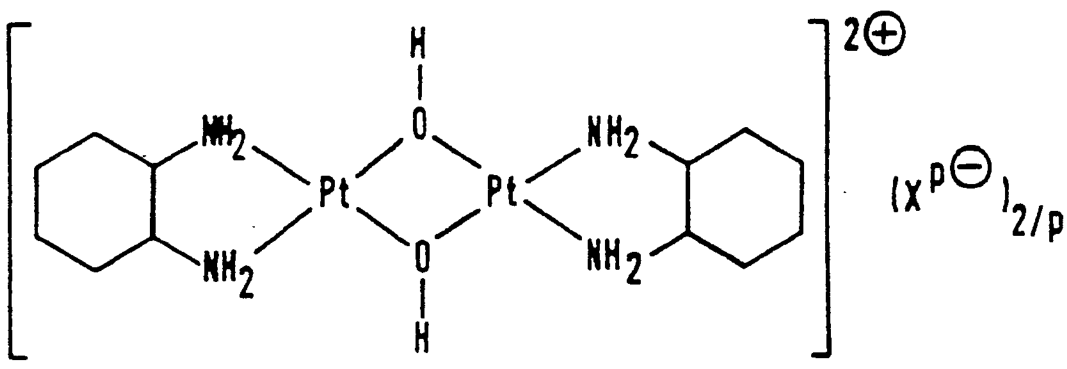

(IIIb)

(in which $X^{p-}$ is as defined above) with the aforementioned compound of formula (V).

Preferred ways of carrying out these reactions are given below as Methods A, B and C.

Method A

Cis-dichloro-(1,2-diaminocyclohexane)platinum (II) is reacted with 2 equivalents of silver nitrate or I equivalent of silver sulphate, to give a compound of formula (IIIa) in which Z and Z′ both represent water molecules and $X^{p-}$ represents a nitrate or sulphate anion, that is to say cis-diaquo-(1,2-diaminocyclohexane)platinum (II) nitrate or sulphate. Corresponding compounds of formula (IIIa) in which $X^{p-}$ represents other anions can be produced by employing another corresponding silver salt. The reaction is preferably carried out in aqueous solution. The reaction temperature is not particularly critical and we therefore find it convenient to carry out the reaction at about room temperature.

The resulting compound of formula (IIIa) is then reacted with the cyclic alkylene urea derivative of formula (V). We prefer to employ from 0.5 to 4 equivalents of said compound of formula (V) per equivalent of said compound of formula (IIIa). The reaction is preferably effected at about neutrality and this may be achieved by adding a suitable alkali. The nature of the alkali is not critical, provided that it does not interfere with the reaction. A suitable alkali is an alkali metal hydroxide, for example sodium hydroxide, which is preferably employed as an approximately IN aqueous solution. The reaction will take place over a wide range of temperatures and the precise temperature chosen is not particularly critical. We prefer to carry out the reaction at a temperature within the range from 0 to 100°C, more preterably from room temperature to 60°C, and preferably with stirring. The time required for the reaction may vary widely, depending upon many factors, notably the nature of the reagents and the reaction temperature. However, at a temperature within the wider range given above, a period of from 1 hour to 40 days will normally suffice, whereas, at a temperature in the more preferred range, a period of from 10 hours to 30 days will normally suffice.

The resulting compound of formula (I) thus obtained may be separated from the reaction mixture by any suitable process. For example, the reaction mixture may be concentrated by evaporation under reduced pressure and then an organic solvent may be added to precipitate the desired compound of formula (I). The nature of the organic solvent is not critical, although we prefer to use a water-miscible organic solvent, such as an alcohol (e.g. methanol, ethanol or isopropanol) or an ether (such as diethyl ether). The resulting precipitate may be collected, for example by filtration, to give the desired compound of formula (I). If required, this precipitate can be further purified by chromatography, for example using an ion-exchange resin, such as Diaion (trade mark) CHP 20P or Sephadex (trade mark).

Method B

An aqueous solution of cis-diaquo-(1,2-diaminocyclohexane)platinum (II) nitrate is prepared as described in Method A and then the water is evaporated off under reduced pressure to give cis-dinitrato-(1,2diaminocyclohexane)platinum (II), i.e. the compound of formula (IIIa) where Z and Z′ both represent nitrate anions. Compounds in which Z and Z′ represent other anions may be prepared correspondingly from salts other than the nitrate. The resulting compound is suspended in water and then reacted with a cyclic alkylene urea (V) in the presence of an alkali (preferably sodium hydroxide) in the manner described in Method A. The compound (V) is preferably employed in an amount of from 0.5 to 4 equivalents and the alkali is preferably employed in an amount of about 1 equivalent per equivalent of the cis-dinitrato-(1,2-diaminocyclohexane)platinum (II).

Method C

An aqueous solution of cis-diaquo-(1,2-diaminocyclohexane)platinum (II) nitrate or sulphate is prepared as described in Method A. An alkaline hydroxide (preferably an alkali metal hydroxide such as sodium hydroxide) is added to this aqueous solution, preferably in an amount of 1 equivalent per equivalent of the nitrate or sulphate. The resulting mixture is then allowed to react to give the corresponding compound of formula (IIIb), i.e. bis [hydroxo-cis-(1,2-diaminocyclohexane)platinum (II)] nitrate or sulphate. The reaction temperature is not particularly critical and we generally prefer to carry out the reaction at about room temperature. The time required for the reaction may vary widely, but a period of from 1 hour to 14 hours will normally suffice. The desired compound of formula (IIIb) is then obtained by evaporating water from the reaction mixture. An aqueous solution of the product is obtained (either by incomplete removal of water of by adding water) and then this is reacted with a cyclic alkylene urea derivative of formula (V), preferably in an amount of from 0.5 to 4 equivalents per equivalent of platinum complex. The reaction will take place over a wide range of temperatures, but we generally find it convenient to employ a temperature within the range from room temperature to 50°C. The time required for the reaction may vary widely, depending upon many factors, but notably the reaction temperature.

A period of from 1 to 20 days will normally suffice. Thereafter, the reaction mixture may be treated as described in Method A, to give the desired compound.

The compounds of the present invention have shown excellent anti-tumour activity which is comparable with or better than that of cisplatin and carboplatin. Moreover, quite unexpectedly, it has been found that the compounds of the invention are even effective against a cisplatin-resistant strain of mouse leukemia L1210. Moreover, the compounds of the invention appear to have surprisingly limited side-effects, such as renal toxicity and bone marrow suppression, and they have a very high solubility in water, which makes them very easy to administer.

There is no particular restriction on the route of administration, but, for use as a carcinostatic agent, the platinum complexes of the present invention are preferably administered parenterally, for example as injections. The dosage may vary depending upon the age, body weight and condition of the patient, as well as the nature and severity of the tumour, but we generally prefer to administer the compound in an amount of from 10 mg to several grams per day for adult human patients, generally as divided doses.

The invention is further illustrated by the following Examples. Preparation of one of the starting materials used in these Examples is described in the following Preparation.

PREPARATION

bis [Hydroxo-cis-(trans-(ℓ)-1,2-diaminocyclohexane)platinum(II)] nitrate

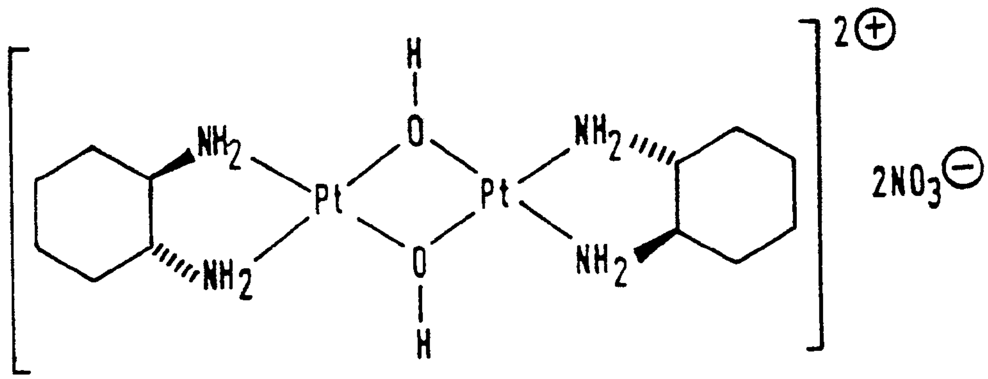

10 g of cis-dichloro-(trans-(ℓ)-1,2diaminocyclohexane)platinum (II) were suspended in 200 ml of water, and then 8.55 g of silver nitrate were added to the suspension. The mixture was stined at zoom temperature for 5 days, and then the insolubles were filtered off with the help of a Celite (trade mark) filter aid. The filtrate, which initially had a pH value of 2.18, was adjusted to a pH value of 7.00 by the addition of a IN aqueous solution of sodium hydroxide. The mixture was then stirred overnight at room temperature, after which water was evaporated off under reduced pressure, until crystals of the title compound were precipitated. The first precipitation yielded 7.28 g of crystals ; these were separated and then evaporation of water was continued to yield a further 2.38 g of crystals.

Nuclear Magnetic Resonance Spectrum (400 MHz, $D_2O$) δ ppm :

0.91 (4H, multiplet) ;
1.03 (4H, multiplet) ;
1.33 (4H, multiplet) ;
1.78 (4H, multiplet) ;
2.0-2.16 (4H, multiplet).

EXAMPLE 1

Hydroxo(ethyleneurea-N,O)-bis [cis-(trans-(ℓ)-, 1,2-diaminocyclohexane)platinum (II)] nitrate

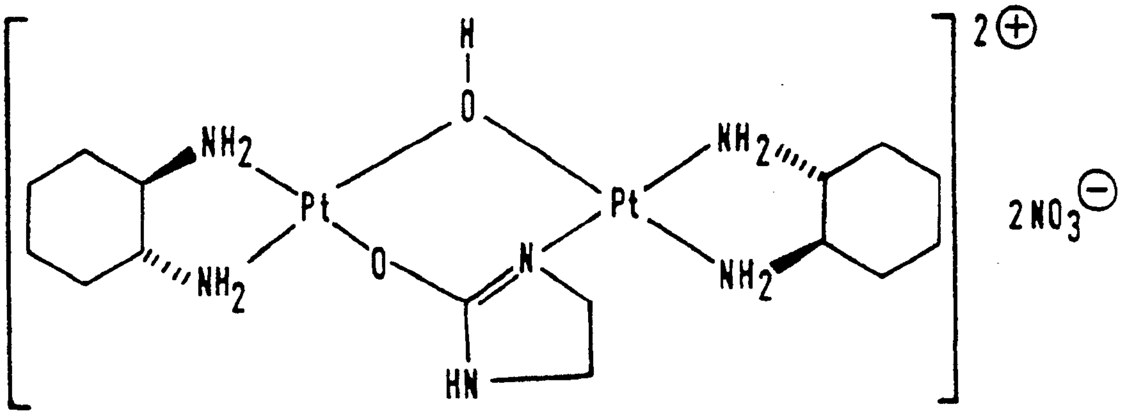

0.871 g of ethyleneurea was added to 270 ml of an aqueous solution containing 5.5 g of bis [hydroxo-cis(trans(ℓ)-1,2-diaminocyclohexane)platinum (II)] nitrate, prepared as described in the Preparation, and the resulting solution was stirred at 28°C for 10 days under a nitrogen stream. At the end of this time, insolubles in the reaction mixture were filtered off and then the water was evaporated off under reduced pressure, to concentrate the aqueous solution to a volume of about 10 ml. 100 ml of ethanol were added to the concentrated aqueous solution and the resulting precipitate was collected by filtration. The whole of this precipitate (1.8 g) was fed to a chromatography column containing about 500 ml of Diaion CHP 20P resin, and the column was eluted with water. The eluate was freeze-dried, to give 0.6 g of the title compound as a colourless powder.

About 400 ml of diethyl ether were added to the filtrate obtained after removing the precipitate of the title compound referred to above. The resulting precipitate (2.4 g) was fed to the same column containing Diaion CHP 20P resin and eluted with water. The eluate was freeze-dried, to give a further 0.8 g of the title compound as a colourless powder.

Nuclear Magnetic Resonance Spectrum (400 MHz, $D_2O$) δ ppm :

0.93 (4H, triplet) ;
1.02 (2H, quartet) ;
1.06 (2H, quartet) ;
1.36 (4H, doublet) ;
1.77-1.83 (4H, multiplet) ;
2.01 (2H, triplet) ;
2.07 (1H, triplet of doublets) ;
2.10 (1H, triplet of doublets) ;
3.17 (2H, multiplet) ;
3.73 (2H, multiplet).
Infrared Absorption Spectrum (KBr) $\nu_{max} cm^{-1}$ :
3430, 3200, 3100, 2940, 1693, 1615.
Mass spectrum (m/z) : 780 ($M-NO_3$), 717 ($780-HNO_3$).

EXAMPLE 2

Hydroxo(ethyleneurea-N,O)-bis [cis-(trans-(d)1,2-diaminocyclohexane)platinum (II)] nitrate

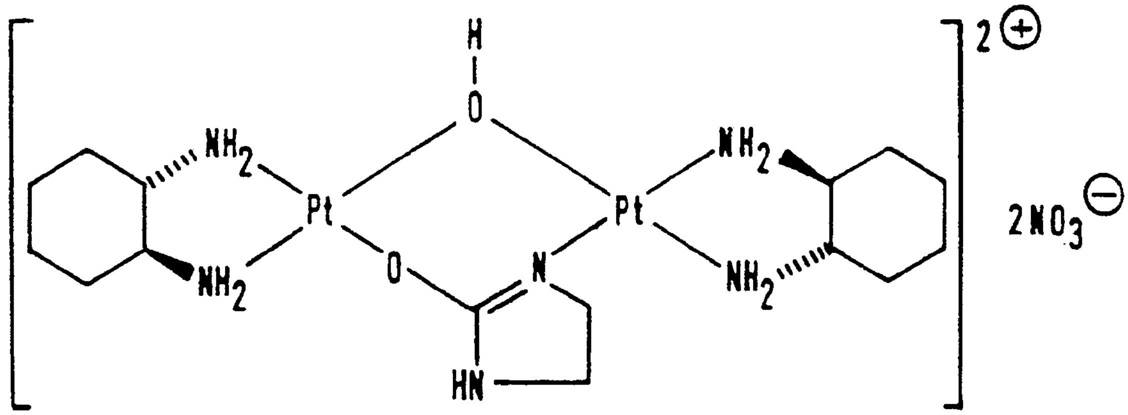

170 ml of an aqueous solution containing 3.43 g of bis[hydroxo-cis-(trans-(d)-1,2-diaminocyclohexane)platinum (II)] nitrate (prepared in a similar manner to that described in the Preparation) and 0.547 g of ethyleneurea were reacted and the product was treated as described in Example 1, to give 0.9 g of the title compound as a colourless powder. The nuclear magnetic resonance and infrared spectra were identical with those of the product obtained as described in Example 1.

EXAMPLE 3

Hydroxo(ethyleneurea-N,O)-bis [cis-(cis-1,2-diaminocyclohexane)platinum (II)] nitrate

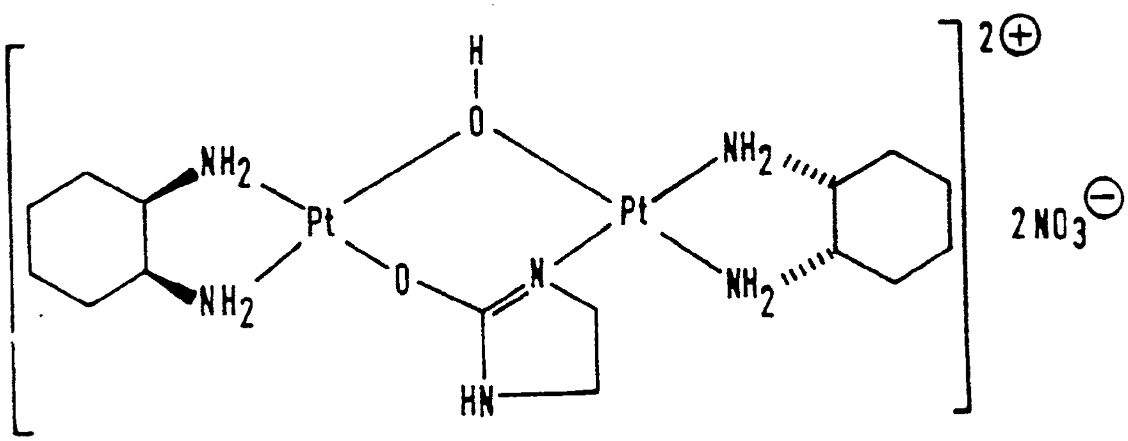

0.39 g of ethyleneurea was added to 200 ml of an aqueous solution containing 2.79 g of bis [hydroxocis-(cis-1,2-diaminocyclohexane)platinum (II)] nitrate (prepared in a similar manner to that described in the Preparation), and the resulting mixture was stirred at 28°C for 13 days under a nitrogen stream. The insolubles in the reaction mixture were filtered off, and then the water was evaporated off under reduced pressure, to concentrate the aqueous solution to a volume of about 100 ml. This concentrated solution was freeze-dried to obtain a powder. 4 ml of water and 100 ml of ethanol were added to the powder and then insolubles were collected by filtration. 600 ml of diethyl ether were added to the filtrate and the resulting precipitate was collected by filtration and combined with the insolubles previously collected. The resulting combined powder was fed to a column containing about 500 ml of Diaion CHP 20P resin, and the column was eluted with water. The resulting eluate was freeze-dried, to give 1 g of the title compound as a colourless powder.

Infrared Absorption Spectrum (KBr) $\nu_{max} cm^{-1}$:

3430, 3200, 1690, 1615.

EXAMPLE 4

Hydroxo(ethyleneurea-N,O)-bis [cis-(trans-(ℓ)-1,2diaminocyclohexane)platinum (II)] nitrate

1.2 g of ethyleneurea was added to 150 ml of an aqueous solution containing 10 mmole of cis-(trans(ℓ)-1,2-diaminocyclohexane)diaquoplatinum (II) nitrate, and then sufficient of a IN aqueous solution of sodium hydroxide was added to adjust the pH of the solution to a value of 7. The solution was then stirred at 28°C for 10 days, after which it was treated in the same manner as described in Example 1, to give 0.8 g of the title compound. The nuclear magnetic resonance and infrared spectra of the resulting product were identical with those of the product obtained as described in Example 1.

EXAMPLE 5

Head-to-head bis(ethyleneurea-N,O)-bis [cis-(trans(ℓ)-1,2-diaminocyclohexane)platinum (II)] nitrate and head-to-tail bis(ethyleneurea-N,O)-bis [cis(trans-(ℓ)-1,2-diaminocyclohexane)platinum (II)] nitrate

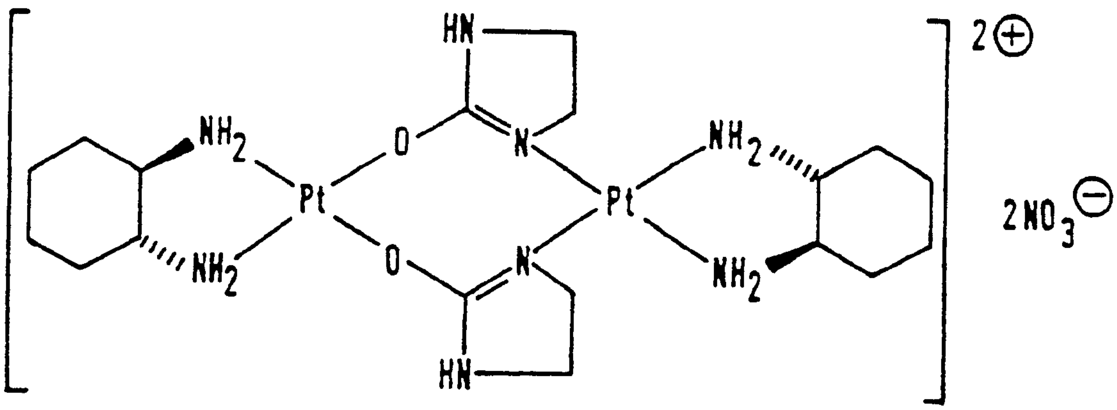

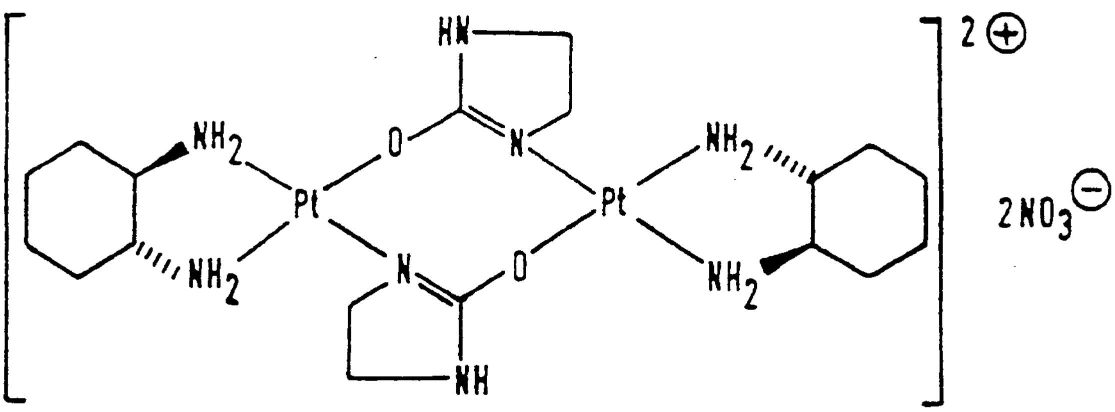

(i) 6.8 g of ethyleneurea were added to 400 ml of an aqueous solution containing 7.51 g of bis [hydroxocis-(trans-(ℓ)-1,2-diaminocyclohexane)platinum (II)] nitrate (prepared as described in the Preparation) and the resulting mixture was stirred, whilst heating it at 50°C, for 16 hours. At the end of this time, the insolubles in the reaction mixture were filtered off, and then the water in the filtrate was evaporated off under reduced pressure, to give about 15 ml of a concentrated solution. About 100 ml of ethanol and about 500 ml of diethyl ether were added to this concentrated solution, and the precipitate which formed was collected by filtration. The precipitate was fed to a chromatography column containing about 1 litre of Diaion CHP 20P resin, and the column was eluted with water. The two isomers of the title compound (the head-to-head and head-to-tail isomers) were eluted separately and the separate eluents were freeze-dried. 0.9 g of isomer (1) were obtained from the first elution, whilst 1.3 g of isomer (2) were obtained from the second elution ; we have not so far identified which isomer is which.

Isomer (1)

Nuclear Magnetic Resonance Spectrum (400 MHz, $D_2O$) δ ppm :

0.93 (4H, triplet) ;
1.03 (2H, quartet) ;
1.06 (2H, quartet) ;
1.37 (4H, doublet) ;
1.80 (4H, doublet) ;
2.05 (4H, multiplet) ;
3.13 (4H, multiplet) ;
3.62 (4H, multiplet).

Isomer (2)

Nuclear Magnetic Resonance Spectrum (400 MHz, $D_2O$) δ ppm :

0.95 (4H, triplet) ;
1.05 (4H, multiplet) ;
1.35 (4H, doublet) ;
1.80 (4H, multiplet) ;
1.99 (1H, multiplet) ;
2.20 (3H, multiplet) ;
3.23 (4H, multiplet) ;
3.90 (4H, multiplet).

(ii) 0.34 g of ethyleneurea was added to 10 ml of an aqueous solution containing 1 mmole of cis-(trans(ℓ)-diaminocyclohexane)diaquoplatinum (II) nitrate, and then sufficient of a IN aqueous solution of sodium hydroxide was added to adjust the pH of the solution to a value of 7. The resulting solution was stirred at 28°C for 1 day and was thereafter stirred at 50°C for 16 hours. The reaction mixture was then treated in the same manner as in Example 5(i) above, to give 0.8 g of isomer (1) and 0.12 g of isomer (2), having the same physical properties as the products of Example 5(i).

EXAMPLE 6

Head-to-head bis(ethyleneurea-N,O)-bis [cis-(trans-(d)1,2-diaminocyclohexane)platinum (II)] nitrate and head-to-tail bis(ethyleneurea-N,O)-bis [cis-(trans-(d)1,2-diaminocyclohexane)platinum (II)] nitrate

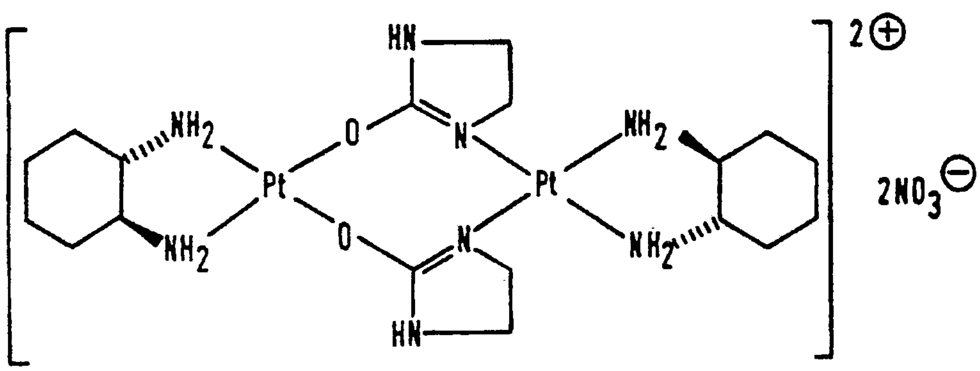

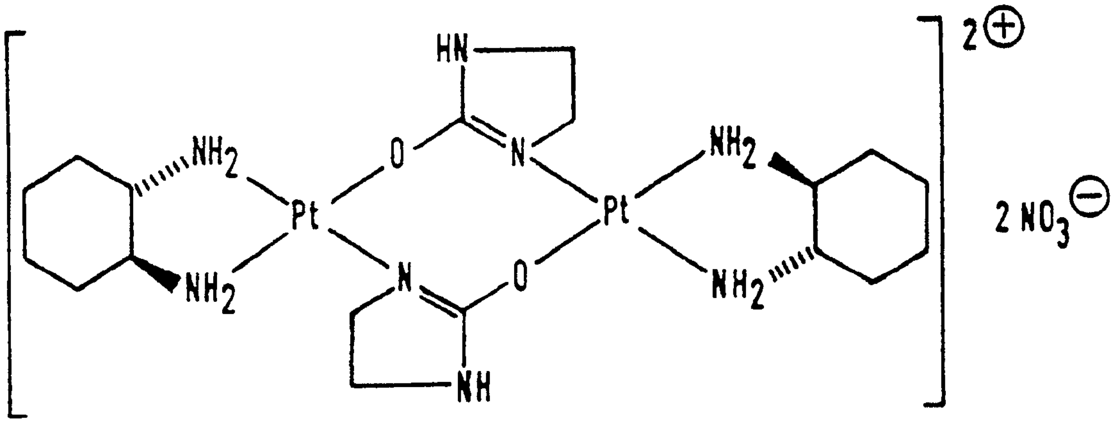

(i) The procedure described in Example 5(i) was repeated, but employing bis [hydroxo-cis-(trans-(d)-1,2diaminocyclohexane)platinum (II)] nitrate as the starting material, to yield two isomers of the title compound, the nuclear magnetic resonance spectra of these isomers being identical with those of the respective isomers obtained as described in example 5(i).

(ii) The procedure described in Example 5(ii) was repeated, but using an aqueous solution containing 1 mmole of cis-(trans-(d)-1,2-diaminocyclohexane)diaquoplatinum (II) nitrate as the starting material, to give the same two isomers as are referred to in Example 6(i) above.

EXAMPLE 7

680 mg of silver nitrate were dissolved in 14 ml of water, and then 760 mg of cis-dichloro(trans-1,2diaminocyclohexane)platinum (II) were added to the solution. The solution was stirred overnight at room temperature, whilst protecting it from light by means of aluminium foil. At the end of this time, the resulting precipitate of silver chloride was filtered off, and then 344 mg of ethyleneurea were added to the resulting filtrate. Sufficient of a IN aqueous solution of sodium hydroxide was then added to the mixture to adjust its pH to a value of 7. The mixture was then stirred at room temperature for 12 hours, after which it was concentrated by evaporation under reduced pressure to give 3 ml of a concentrated aqueous solution. 70 ml of ethanol and 60 ml of diethyl ether were added to this concentrated solution, and the precipitate which formed was collected by filtration to give 435 mg of a platinum-ethyleneurea complex as a colourless powder.

This complex is a mixture of the dl-compound corresponding to the compound of Example 1 and the dl-compounds corresponding to the two isomers of the compound in Example 5.

Infrared Absorption Spectrum (KBr) $\nu_{max}cm^{-1}$ :

3200, 3100, 1690, 1630, 1390, 1130.

EXAMPLE 8

312 mg of silver sulphate and 380 mg of cis-dichloro(trans-1,2-diaminocyclohexane)platinum (II) were suspended in 7 ml of water, and the suspension was stirred over an oil bath maintained at a bath temperature of 50°C for 4 days, whilst protecting the reaction mixture from light by means of aluminium foil. The silver chloride which precipitated was filtered off, and then 172 mg of ethyleneurea were added to the filtrate. Sufficient of a IN aqueous solution of sodium hydroxide was added to adjust the pH of the reaction mixture to a value of 7. The mixture was then stirred at 50°C for 10 hours. At the end of this time, the reaction mixture was concentrated by evaporation under reduced pressure, to give about 2 ml of a concentrated aqueous solution. 15 ml of ethanol and 50 ml of diethyl ether were added to this concentrated solution, to form an oily precipitate. Isopropanol was added to the oily precipitate and then the resulting powdery precipitate was collected by filtration, to give 376 mg of the sulphate of an ethyleneurea-platinum complex, as a colourless powder. This complex is a mixture of the sulphates of the dl-compound corresponding to the compound of Example 1 and of the dl-compounds corresponding to the two isomers of the compound of Example 5.

Infrared Absorption Spectrum (KBr) $\nu_{max}cm^{-1}$

3200, 3080, 1690, 1630, 1180.

EXAMPLE 9

Hydroxo(propyleneurea-N,O)-bis [cis-(trans-(d)-1,2diaminocyclohexane)platinum (II)] nitrate

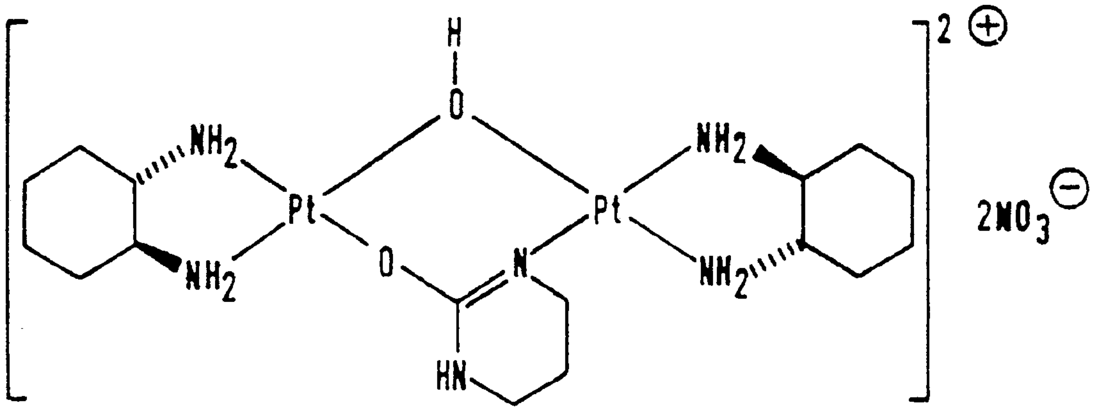

100 mg of propyleneurea were added to an aqueous solution containing 390 mg of bis [hydroxo-cis-(trans(d)-1,2-diaminocyclohexane)platinum (II)] nitrate (prepared in a manner similar to that described in the Preparation), and the resulting mixture was allowed to stand at 40°C for 1 month. At the end of this time, water was distilled from the reaction mixture under reduced pressure, and then the residue was charged into a chromatography column containing about 50 ml of Diaion CHP 20P resin. The column was then eluted with water, and the eluent was freeze-dried, to give 302 mg of the title compound.

Nuclear Magnetic Resonance Spectrum (270 MHz, $D_2O$) δ ppm :

0.8-1.3 (8H, multiplet) ;
1.3-1.6 (8H, multiplet) ;
1.75-2.0 (4H, multiplet) ;
2.2-2.45 (2H, multiplet) ;
2.9-3.0 (2H, multiplet) ;
3.4-3.5 (2H, triplet).

BIOLOGICAL ACTIVITY

In the following experiments, the test animals used were 8 to 9 week old female mice of the $CDF_1$ strain, each weighing 20-25 g. The standard L1210 leukemia cells were supplied by Dr. T. Yamamoto of the Institute of Medical Science, University of Tokyo, Japan, whilst the cisplatin-resistant L1210 cell line (L1210/CDDP) was supplied by Dr. T. Tashiro of the Cancer Chemotherapy Centre, Japanese Foundation for Cancer Research, Tokyo, Japan. The degree of cisplatin resistance of the cisplatin-resistant cell line in terms of in vitro sensitivity was 40-fold that of the parent L1210 cell line.

EXPERIMENT 1

$CDF_1$ mice were inoculated intraperitoneally with L1210 cells ($10^5$ cells/mouse). The compound of the present invention (that prepared as described in Example 1) and carboplatin were each dissolved in 5% v/v aqueous mannitol, whilst cisplatin was dissolved in a 5% v/v solution of mannitol in physiological saline. Each drug was injected intraperitoneally on days 1 and 4 following tumour implantation. The number of mice in each test group was 6.

The increase in life span (ILS) was calculated as follows :

$$ILS\% = [(S_t/S_u) - 1] \times 100$$

in which :

$S_t$ = weighted median number of days survival of treated mice ; and
$S_u$ = weighted median number of days survival of untreated mice.

The results are shown in Table 1.

EXPERIMENT 2

The procedures described in Experiment 1 were repeated exactly, except that the tumour cells were cisplatin-resistant L1210/CDDP cells. The results are shown in Table 2.

Table 1

| Compound | Dose (mg/kg) | Wt. Change (g) 1) | Med.S.T. (day) 2) | ILS (%) 3) | Survivors on day 42 |
|---|---|---|---|---|---|
| Untreated Control | - | +1.9 | 8.4 | - | 0/6 |
| Compound of Example 1 | 5 | +2.6 | 9.0 | 7 | 0/6 |
| | 10 | +2.8 | 11.0 | 31 | 0/6 |
| | 20 | +2.2 | 16.0 | 90 | 0/6 |
| | 40 | +0.4 | 35.0 | 317 | 2/6 |
| | 80 | +0.2 | >41.7 | >396 | 3/6 |
| | 160 | -3.0 | >41.7 | >396 | 3/6 |
| | 240 | - (2/6, toxic) | >41.9 | >399 | 4/6 |
| | 320 | - (5/6, toxic) | 4.8 | -43 | 0/6 |
| carboplatin | 5 | +1.3 | 9.0 | 7 | 0/6 |
| | 10 | +2.0 | 9.0 | 7 | 0/6 |
| | 20 | +0.8 | 9.0 | 7 | 0/6 |
| | 40 | +0.4 | 9.2 | 10 | 0/6 |
| | 80 | -0.5 | 10.3 | 23 | 0/6 |

Table 1 (cont)

| Compound | Dose (mg/kg) | Wt. Change (g) 1) | Med.S.T. (day) 2) | ILS (%) 3) | Survivors on day 42 |
|---|---|---|---|---|---|
| cisplatin | 0.625 | +1.6 | 9.1 | 8 | 0/6 |
| | 1.25 | +2.5 | 9.7 | 15 | 0/6 |
| | 2.5 | +1.7 | 13.3 | 58 | 0/6 |
| | 5 | -3.5 | 39.0 | 364 | 2/6 |
| | 10 | -5.5 (6/6, toxic) | 10.3 | 23 | 0/6 |

Notes:

1) Change in body weight from day 1 to day 7.

2) Median survival time (days).

3) Increase in life span.

Table 2

| Compound | Dose (mg/kg) | Body weight change from days 1 to 7 | Median survival days | ILS(%) | 30-day survivors |
|---|---|---|---|---|---|
| Untreated control | - | +2.6 | 9.1 | - | 0/12 |
| Compound of Example 1 | 5 | +2.4 | 10.8 | 19 | 0/6 |
| | 10 | +2.3 | 13.3 | 46 | 1/6 |
| | 20 | +2.2 | 20.0 | 120 | 2/6 |
| | 40 | -0.3 | >30.0 | >230 | 6/6 |
| | 80 | -0.5 | >30.0 | >230 | 6/6 |
| | 160 | -1.8 | >30.0 | >230 | 6/6 |
| carboplatin | 30 | +1.2 | 9.9 | 9 | 0/6 |
| | 60 | -0.6 | 9.8 | 8 | 0/6 |
| cisplatin | 2.5 | +1.0 | 9.3 | 2 | 0/5 |
| | 5.0 | -1.9 | 9.9 | 9 | 0/6 |

## Claims

**Claims for the contracting States BE, CH, DE, FR, GB, IT, LI, LU, NL, SE :**

1. Compounds of formula (I) :

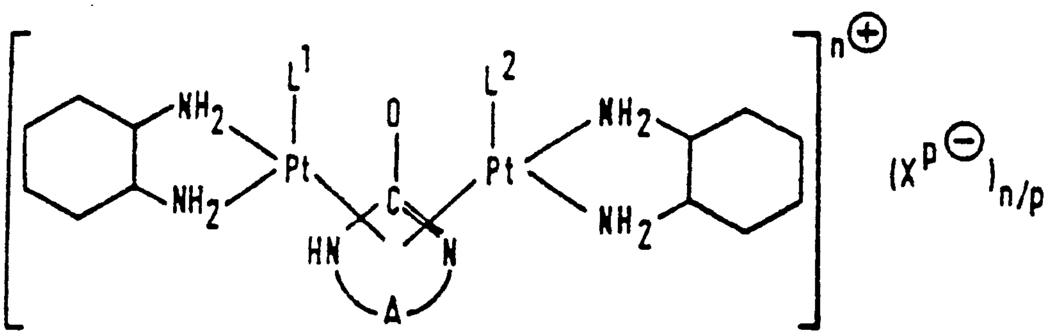

(I)

wherein :

A represents an alkylene group having 2 or 3 carbon atoms ;

$L^1$ and $L^2$ are the same or different and each represents water, a nitrate ion, a halide ion, a perchlorate ion, a sulphate ion, a carbonate ion, a hydroxide ion, a methoxide ion, or an ethoxide ion, or $L^1$ and $L^2$ together represent a nitrate ion, chloride ion, sulphate ion, hydroxide ion, methoxide ion, ethoxide ion, perchlorate ion, or carbonate ion, or a group of formula (II) :

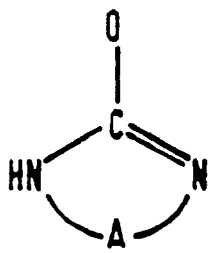

(II)

where A is as defined above ;

$X^{p-}$ represents an anion of valency p, being a nitrate ion, perchlorate ion, sulphate ion or carbonate ion and

n is 0, 1 or 2.

2. Compounds as claimed in Claim 1, wherein said neutral ligand is water.

3. Compounds as claimed in Claim 1 or 2, wherein $X^{p-}$ represents a nitrate ion or a sulphate ion.

4. Compounds as claimed in Claim 1, wherein $L^1$ and $L^2$ together represent a hydroxide ion

5. Compounds as claimed in Claim 1, wherein $L^1$ and $L^2$ together represent said group of formula (II).

6. Hydroxo(ethyleneurea-N,O)-bis [cis-(trans-(ℓ)1,2-diaminocyclohexane)platinum (II)] nitrate.

7. Hydroxo(ethyleneurea-N,O)-bis(cis-(trans-(d)-1,2-diaminocyclohexane)platinum (II)] nitrate.

8. Hydroxo(ethyleneurea-N,O)-bis [cis-(cis-1,2-diaminocyclohexane)platinum (II)] nitrate.

9. Bis(ethyleneurea-N,O)-bis [cis-(trans-(ℓ)-1,2-diaminocyclohexane)platinum (II)] nitrate.

10. Bis(ethylneurea-N,O)-bis [cis-(trans-(d)-1,2-diaminocyclohexane)platinum (II)] nitrate.

11. Hydroxo(propyleneurea-N,O)-bis [cis-trans-(d-)-1,2-diaminocyclohexane)platinum (II)] nitrate.

12. A process for preparing a compound as claimed in any one of Claims 1 to 14, which process comprises reacting a platinum complex of formula (III) :

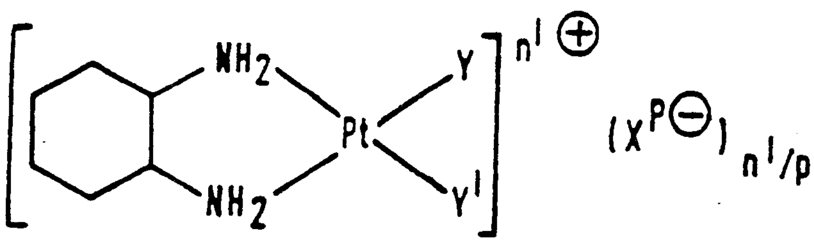

(III)

in which :

$X^{p-}$ is as defined in Claim 1 ;

n' is 0, 1 or 2 ; and

Y and Y' are the same or different and each represents water, a nitrate ion, a hydroxide ion or a perchlorate ion or Y and Y' are linked together to represent a sulphate or carbonate ion or a group of formula (IV) :

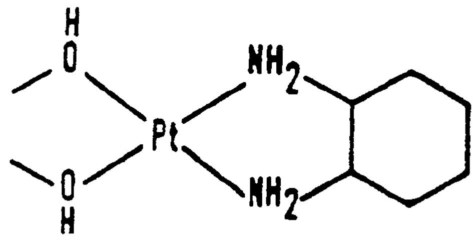

(IV)

with a compound of formula (V) :

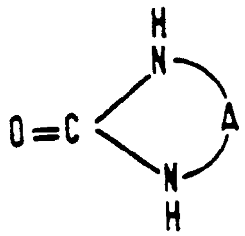

(V)

(in which A is as defined in Claim 1).

13. A pharmaceutical composition comprising an anti-tumour agent and a pharmaceutically acceptable carrier or diluent, wherein the anti-tumour agent is at least one compound as claimed in any one of Claims 1 to 11.

14. The use for the manufacture of a medicament for the treatment of an animal suffering from a tumour of a compound as claimed in any one of Claims 1 to 11.

**Claims for the Contracting State AT :**

1. A process for preparing a compound of formula (I) :

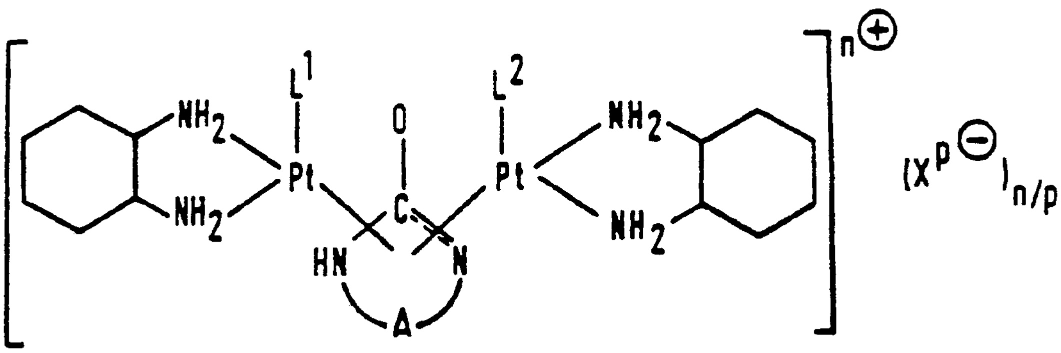

(I)

[wherein :

A represents an alkylene group having 2 or 3 carbon atoms ;

$L^1$ and $L^2$ are the same or different and each represents water, a nitrate ion, a halide ion, a perchlorate ion, a sulphate ion, a carbonate ion, a hydroxide ion, a methoxide ion, or an ethoxide ion, or $L^1$ and $L^2$ together represent a nitrate ion, chloride ion, sulphate ion, hydroxide ion, methoxide ion, ethoxide ion, perchlorate ion, or carbonate ion, or a group of formula (II) :

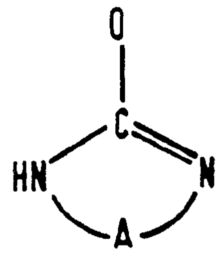

(II)

where A is as defined above ;

$X^{p-}$ represents an anion of valency p ; being a nitrate ion, perchlorate ion, sulphate ion or carbonate ion.

n is 0, 1 or 2],

which process comprises reacting a platinum complex of formula (III) :

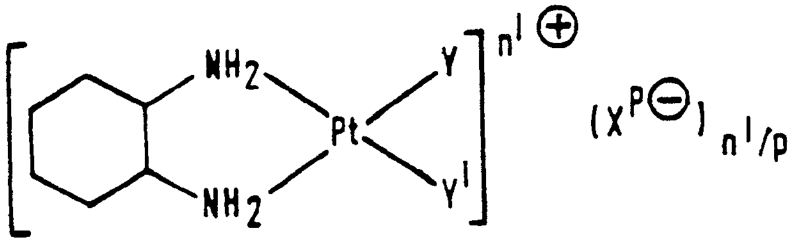

(III)

[in which :

$X^{p-}$ is as defined above ;

n' is 0, 1 or 2 ; and

Y and Y' are the same or different and each represents water, a nitrate ion, a hydroxide ion or a perchlorate ion or Y and Y' are linked together to represent a sulphate or carbonate ion or a group of formula (IV) :

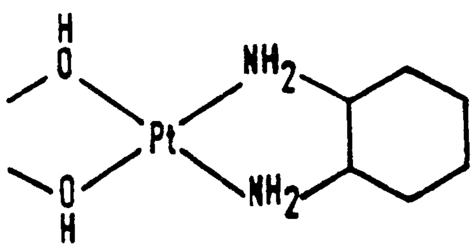

(IV)]

with a compound of formula (V) :

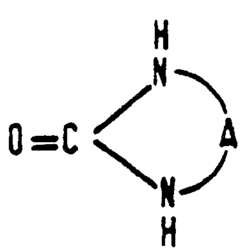

(V)

(in which A is as defined above).

2. A process as claimed in Claim 1, wherein said reaction is carried out in an aqueous medium to prepare a compound where said neutral ligand $L^1$ and/or $L^2$ is water.

3. A process as claimed in Claim 1 or 2, wherein $X^{p-}$ represents a nitrate ion or a sulphate ion.

4. A process as claimed in Claim 1, wherein $L^1$ and $L^2$ together represent a hydroxide ion.

5. A process as claimed in Claim 1, wherein $L^1$ and $L^2$ together represent said group of formula (II).

6. A process according to Claim 1, where the reagents and reaction conditions are so chosen as to prepare:
hydroxo(ethyleneurea-N,O)-bis [cis-(trans-(ℓ)-1,2-diaminocyclohexane)platinum (II)] nitrate ;
hydroxo(ethyleneurea-N,O)-bis [cis-(trans-(d)-1,2-diaminocyclohexane)platinum (II)] nitrate ;
hydroxo(ethyleneurea-N,O)-bis [cis-(cis-1,2-diaminocyclohexane)platinum (II)] nitrate ;
bis(ethyleneurea- N,O)-bis [cis-(trans-(ℓ)-1,2-diaminocyclohexane)platinum (II)] nitrate ;
bis(ethyleneurea- N,O)-bis [cis-(trans-(d)-1,2-diaminocyclohexane)platinum (II)] nitrate ; or
hydroxo(propyleneurea-N,O)-bis [cis-(trans-(d)-1,2-diaminocyclohexane)platinum (II)] nitrate.

7. The use for the manufacture of a medicament for the treatment of an animal suffering from a tumour of a compound of formula (I), wherein the formula is as defined in Claim 1.

## Revendications

### Revendications pour les Etats Contractants : BE, CH, DE, FR, GB, IT, LI, LU, NL, SE.

1. Composés de formule (I) :

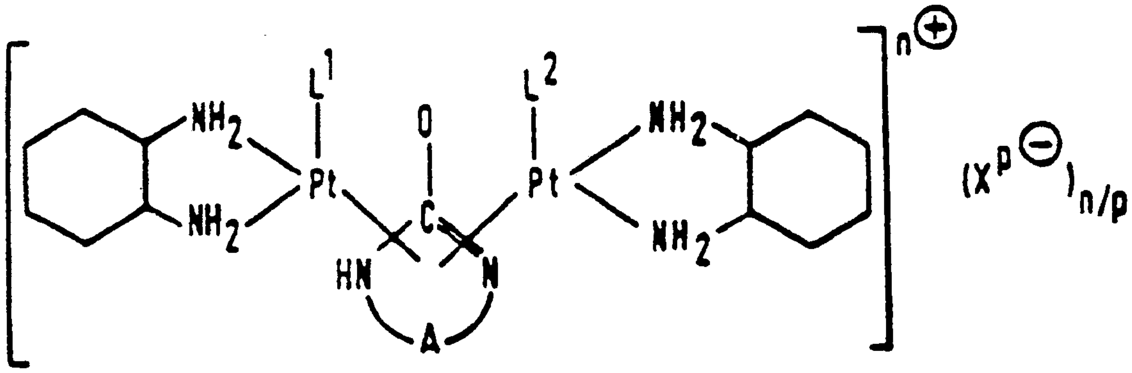

(I)

dans laquelle :

A représente un groupe alkylène comprenant deux ou trois atomes de carbone ;

$L^1$ et $L^2$ sont identiques ou différents, et ils représentent chacun l'eau, un ion nitrate, halogénure, perchlorate, sulfate, carbonate, hydroxyde, méthylate ou éthylate, ou $L^1$ et $L^2$ représentent ensemble un ion nitrate, chlorure, sulfate, hydroxyde, méthylate, éthylate, perchlorate ou carbonate, ou un groupe de formule (II) :

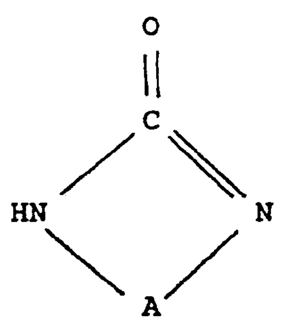

(II)

dans laquelle A est tel que défini ci-dessus ;

$X^{p-}$ représente un anion de valence p, comprenant un ion nitrate, perchlorate, sulfate ou carbonate ;

et n est égal à 0, 1 ou 2.

2. Composés selon la revendication 1, dans lesquels ledit ligand neutre est l'eau.

3. Composés selon la revendication 1 ou 2, dans lesquels $X^{p-}$ représente un ion nitrate ou sulfate.

4. Composés selon la revendication 1, dans lesquels $L^1$ et $L^2$ représentent ensemble un ion hydroxyde.

5. Composés selon la revendication 1, dans lesquels $L^1$ et $L^2$ représentent ensemble ledit groupe de formule (II).

6. Nitrate d'hydroxo(éthylèneurée-N,O)-bis [cis(trans-(l)-1,2-diéminocyclohexane)platine (II).

7. Nitrate d'hydroxo(éthylèneurée-N,O)-bis [cis- (trans-(d)-1,2-diaminocyclohexane)platine (II)].

8. Nitrate d'hydroxo(éthylèneurée-N,O)-bis [cis(cis-1,2-diaminocyclohexane)platine (II)].

9. Nitrate de bis(éthylèneurée-N,O)-bis [cis(trans-(l)-1,2-diaminocyclohexane)platine (II)].

10. Nitrate de bis(éthylèneurée-N,O)-bis [cis(trans-(d)-1,2-diaminocyclohexane)platine (II)].

11. Nitrate d'hydroxo(propylèneurée-N,O)bis [cis-(trans-(d)-1,2-diaminocyclohexane)platine (II)].

12. Procédé de préparation d'un composé selon l'une quelconque des revendications précédentes, selon lequel on fait réagir un complexe dérivé du platine de formule (III) :

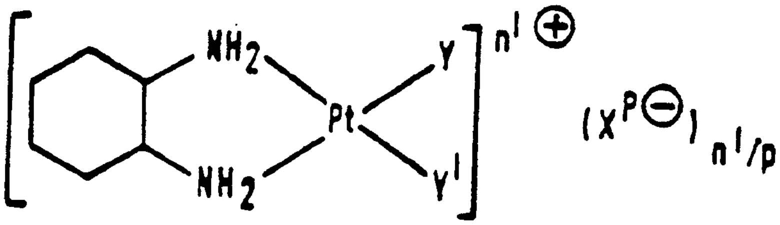

(III)

dans laquelle :

$X^{p-}$ est tel que défini dans la revendication 1 ;

n' est égal à 0, 1 ou 2 ; et

Y et Y' sont identiques ou différents, et ils représentent chacun l'eau, un ion nitrate, hydroxyde ou perchlorate, ou Y et Y' sont liés ensemble et représentent un ion sulfate ou carbonate, ou un groupe de formule (IV) :

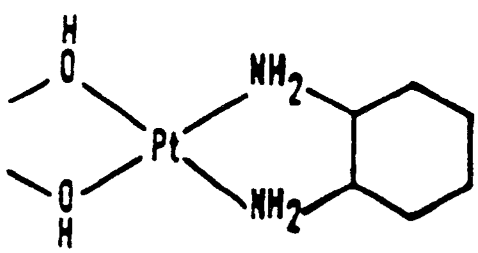

(IV)

avec un composé de formule (V) :

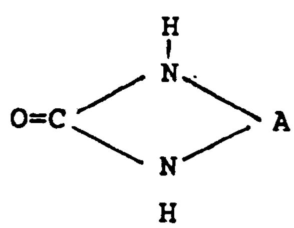

(V)

dans lequel A est tel que défini dans la revendication 1.

13. Composition pharmaceutique comprenant un agent antitumoral, et un véhicule ou un diluant pharmaceutiquement acceptable, dans laquelle l'agent antitumoral consiste en au moins un composé selon l'une quelconque des revendications 1 à 11.

14. Utilisation d'un composé selon l'une quelconque des revendications 1 à 11, pour la fabrication d'un médicament pour le traitement d'un animal souffrant d'une tumeur.

**Revendications pour l'Etat Contractant : AT**

1. Procédé de préparation d'un composé de formule (I) :

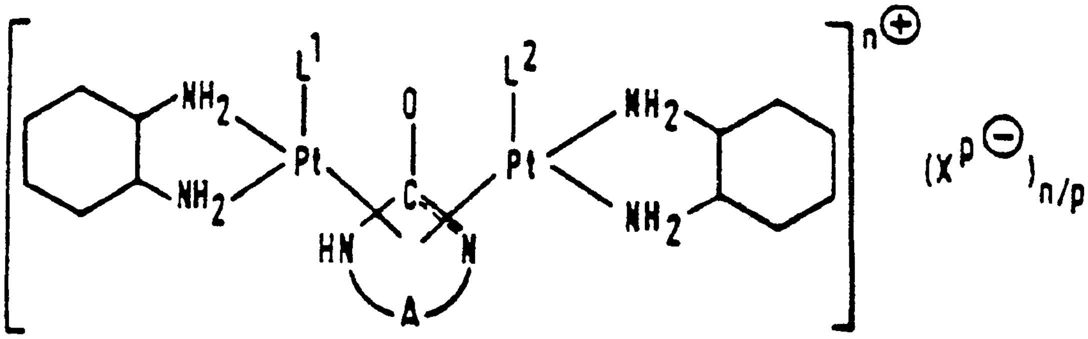

(I)

dans laquelle :

A représente un groupe alkylène comportant deux ou trois atomes de carbone ;

$L^1$ et $L^2$ sont identiques ou différents, et ils représentent chacun l'eau, un ion nitrate, halogénure, perchlorate, sulfate, carbonate, hydroxyde, méthylate ou éthylate, ou $L^1$ et $L^2$ représentent ensemble un ion nitrate, chlorure, sulfate, hydroxyde, méthylate, éthylate, perchlorate ou carbonate, ou un groupe de formule (II) :

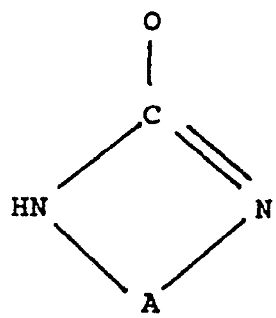

(II)

dans laquelle A est tel que défini ci-dessus ;

$X^{p-}$ représente un anion de valence p, comprenant un ion nitrate, perchlorate, sulfate ou carbonate ;

n est égal à 0, 1 ou 2, selon lequel on fait réagir un complexe dérivé du platine de formule (III) :

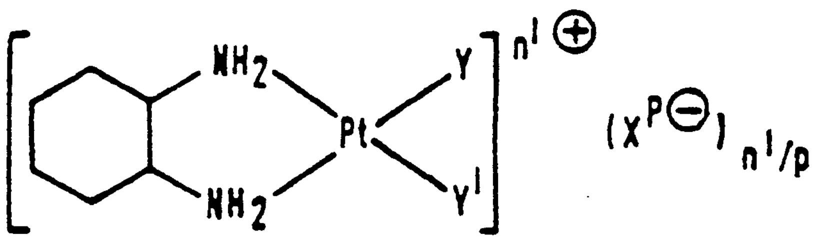

(III)

dans laquelle :

$X^{p-}$ est tel que défini ci-dessus ;

n' est égal à 0, 1 ou 2 ; et

Y et Y' sont identiques ou différents, et ils représentent chacun l'eau, un ion nitrate, hydroxyde ou perchlorate, ou Y et Y' sont liés ensemble et représentent un ion sulfate ou carbonate, ou un groupe de formule (IV):

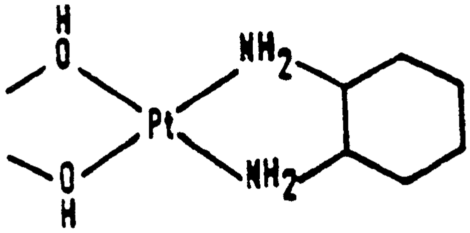

(IV)

avec un composé de formule (V) :

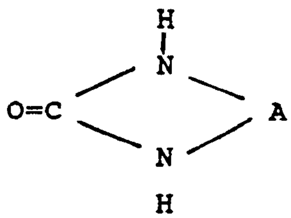

(V)

dans lequel A est tel que défini dans la revendication 1.

2. Procédé selon la revendication 1, dans lequel la réaction est effectuée dans un milieu aqueux afin de préparer un composé dans lequel les ligands neutres $L^1$ et/ou $L^2$, consistent en eau.

3. Procédé selon la revendication 1 ou 2, dans lequel $X^{p-}$ représente un ion nitrate ou sulfate.

4. Procédé selon la revendication 1, dans lequel $L^1$ et $L^2$ représentent ensemble un ion hydroxyde.

5. Procédé selon la revendication 1, dans lequel $L^1$ et $L^2$ représentent ensemble le groupe de formule (II).

6. Procédé selon la revendication 1, dans lequel les réactifs et les conditions réactionnelles sont choisis de façon à préparer le nitrate d'hydroxo(éthylèneurée-N,O)-bis [cis-(trans-(l)-1,2diaminocyclohexane)platine (II) ;

le nitrate d'hydroxo(éthylèneurée-N,O)-bis [cis(trans-(d)-1,2-diaminocyclohexane)platine (II)] ;

le nitrate d'hydroxo(éthylèneurée-N,O)-bis [cis(cis-1,2-diaminocyclohexane)platine (II)] ;

le nitrate de bis(éthylèneurée-N,O)-bis [cis(trans-(1)-1,2-diaminocyclohexane)platine (II)] ;

le nitrate de bis(éthylèneurée-N,O)-bis [cis(trans-(d)-1,2-diaminocyclohexane)platine (II)] ; ou

le nitrate d'hydroxo(propylèneurée-N,O)-bis [cis(trans-(d)-1,2-diaminocyclohexane)platine (II)].

7. Utilisation d'un composé de formule (I) défini dans la revendication 1, pour la fabrication d'un médicament pour le traitement d'un animal souffrant d'une tumeur.

**Ansprüche**

**Patentansprüche für die bennanten Vertragsstaaten : BE, CH, DE, FR, GB, IT, LI, LU, NL, SE.**

1. Verbindungen der Formel(I) :

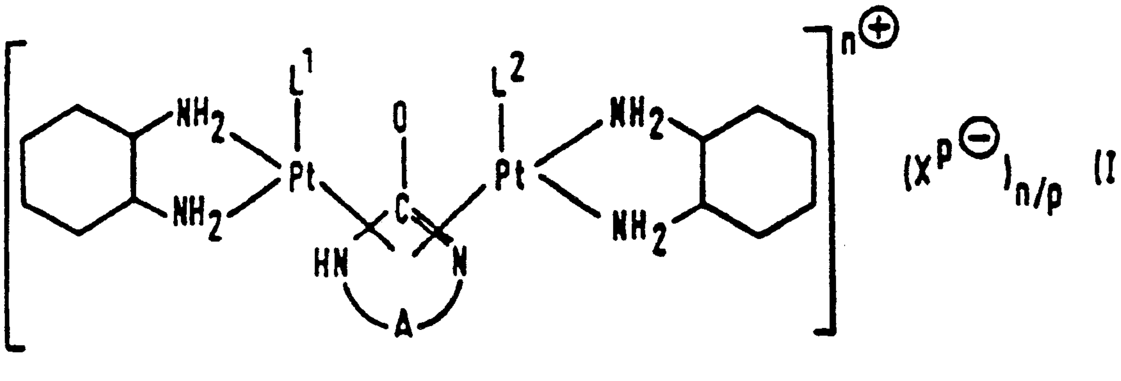

(I)

worin :

A eine Alkylengruppe mit 2 oder 3 Kohlenstoffatomen bedeutet ;

$L^1$ und $L^2$ untereinander gleich oder voneinander verschieden sind und je für sich Wasser, ein Nitration, ein Halogenion, ein Perchloration, ein Sulfation, ein Carbonation, ein Hydroxidion, ein Methoxidion oder ein Äthoxidion bedeuten oder $L^1$ und $L^2$ gemeinsam ein Nitration, Chloridion, Sulfation, Hydroxidion, Methoxidion, Äthoxidion, Perchloration oder Carbonation oder eine Gruppe der Formel (II) :

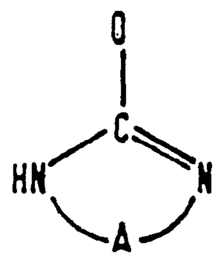

(II)

mit der oben angegebenen bedeutung für A bilden ;

$X^{p\ominus}$ ein Anion der Wertigkeit p ist und ein Nitration, Perchloration, Sulfation oder Carbonation darstellt und n gleich ist 0, 1 oder 2.

2. Verbindungen nach Anspruch 1, worin der neutrale Ligand Wasser ist.

3. Verbindungen nach Anspruch 1 oder 2, worin $X^{p\ominus}$ ein Nitration oder Sulfation darstellt.

4. Verbindungen nach Anspruch 1, worin $L^1$ und $L^2$ gemeinsam ein Hydroxidion bilden.

5. Verbindungen nach Anspruch 1, worin $L^1$ und $L^2$ gemeinsam die Gruppe der Formel (II) bilden.

6. Hydroxo(äthylharnstoff-N,O)-bis [cis-(trans-(l)-1,2-diaminocyclohexan)platin(II)] nitrat.

7. Hydroxo(äthylharnstoff-N,O)-bis(cis-(trans-(d)-1,2--diaminocyclohexan)platin(II)] nitrat.

8. Hydroxo(äthylharnstoff-N,O)-bis [cis-(cis-1,2--diaminocyclohexan)platin(II)] nitrat.

9. Bis(äthylharnstoff-N,O)-bis [cis-(trans-(1)-1,2--diaminocyclohexan)platin(II)] nitrat.

10. Bis(äthylharnstoff-N,O)-bis [cis-(trans-(d)-1,2--diaminocyclohexan)platin(II)] nitrat.

11. Hydroxo(propylharnstoff-N,O)-bis [cis-(trans-(d)-1,2-diaminocyclohexan)platin(II)] nitrat.

12. Verfahren zum Herstellen einer in irgendeinem der Ansprüche 1 bis 11 beanspruchten Verbindung, bei welchem ein Platinkomplex der Formel (III) :

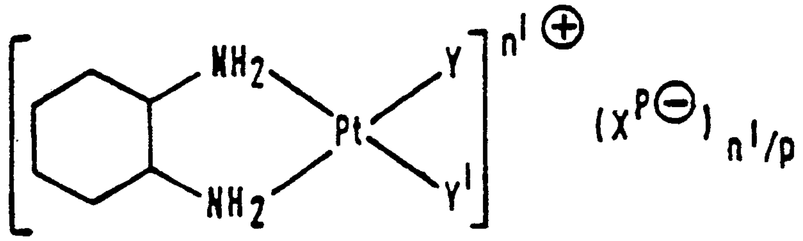

(III)

in welcher

$X^{p\ominus}$ die in Anspruch 1 angegebene Bedeutung besitzt,

n' gleich ist 0, 1 oder 2 und

Y und Y' untereinander gleich oder voneinander verschieden sind und je für sich Wasser, ein Nitration, ein Hydroxidion oder ein Perchloration bedeuten oder Y und Y' miteinander verbunden sind und ein Sulfation oder Carbonation oder eine Gruppe der Formel (IV) :

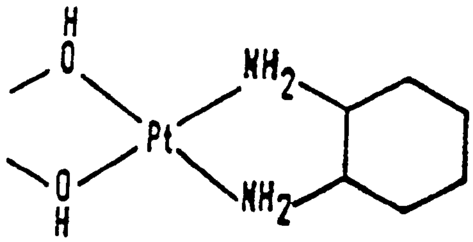

(IV)

bilden, mit einer Verbindung der Formel (V) :

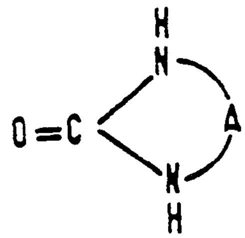

(V)

(in welcher A die in Anspruch 1 angegebene Bedeutung besitzt) umgesetzt wird.

13. Pharmazeutische Mischung, welche ein Antitumormittel und einen pharmazeutisch annehmbaren Träger oder ein pharmazeutisch annehmbares Verdünnungsmittel enthält, worin das Antitumormittel zumindest eine in irgendeinem der Ansprüche 1 bis 11 beanspruchte Verbindung ist.

14. Verwendung einer in irgendeinem der Ansprüche 1 bis 11 beanspruchten Verbindung für die Herstellung eines Heilmittels zum Behandeln eines an einem Tumor leidenden Tieres.

**Patentansprüche für den bennanten Vertragsstaat : AT**

1. Verfahren zum Herstellen einer Verbindung der Formel (I) :

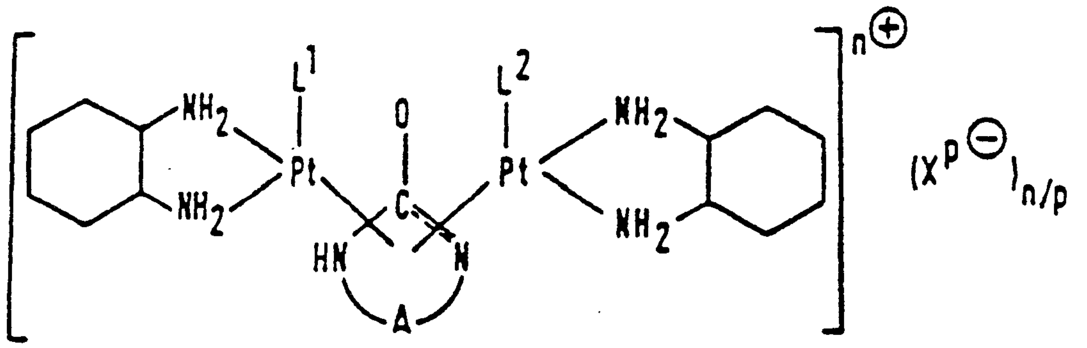

(I)

[worin :

A eine Alkylengruppe mit 2 oder 3 Kohlenstoffatomen bedeutet :

$L^1$ und $L^2$ untereinander gleich oder voneinander verschieden sind und je für sich Wasser, ein Nitration, ein Halogenion, ein Perchloration, ein Sulfation, ein Carbonation, ein Hydroxidion, ein Methoxidion oder ein Äthoxidion bedeuten oder $L^1$ und $L^2$ gemeinsam ein Nitration, Chloridion, Sulfation, Hydroxidion, Methoxidion, Äthoxidion, Perchloration oder Carbonation oder eine Gruppe der Formel (II) :

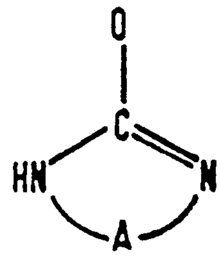

(II)

mit der oben angegebenen Bedeutung für A bilden ;

$X^{p\ominus}$ ein Anion der Wertigkeit p ist und ein Nitration, Perchloration, Sulfation oder Carbonation darstellt und n gleich ist 0, 1 oder 2].

bei welchem Verfahren ein Platinkomplex der Formel (III) :

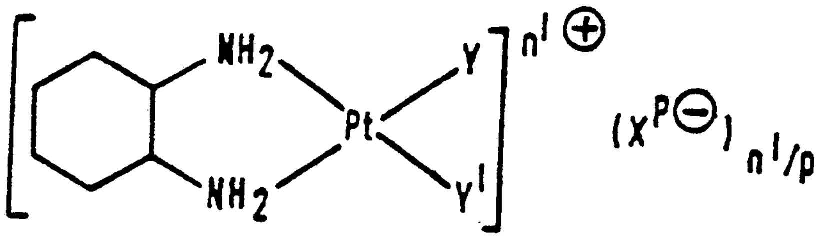

(III)

[in welcher

$X^{p\ominus}$ die oben angegebene Bedeutung besitzt,

n' gleich ist 0, 1 oder 2 und

Y und Y' untereinander gleich oder voneinander verschieden sind und je für sich Wasser, ein Nitration, ein Hydroxidion oder ein Perchloration bedeuten oder Y und Y' miteinander verbunden sind und ein Sulfation oder Carbonation oder eine Gruppe der Formel (IV) :

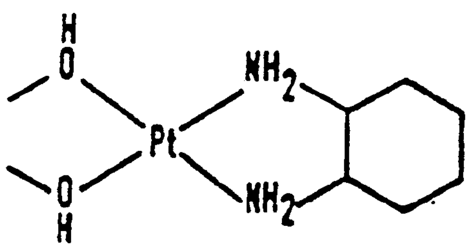

(IV)]

bilden],

mit einer Verbindung der Formel (V) :

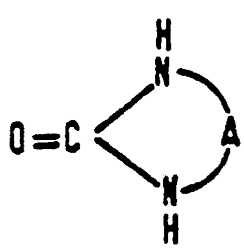

(V)

(in welcher A die oben angegebene Bedeutung besitzt), umgesetzt wird,

2. Verfahren nach Anspruch 1, worin die Umsetzung in einem wässerigen Medium durchgeführt wird, um eine Verbindung herzustellen, in welcher der neutrale Ligand $L^1$ und/oder $L^2$ Wasser ist.

3. Verfaren nach Anspruch 1 oder 2, worin $X^{p\ominus}$ ein Nitration oder ein Sulfation bedeutet.

4. Verfahren nach Anspruch 1, worin $L^1$ und $L^2$ gemeinsam ein Hydroxidion bilden.

5. Verfahren nach Ansprüch 1, worin $L^1$ und $L^2$ gemeinsam die Gruppe der Formel (II) bilden.

6. Verfahren nach Anspruch 1, worin die Reaktionsteilnehmer und die Umsetzungsbedingungen so gewählt werden, daß

Hydroxo(äthylharnstoff N,O)-bis [cis-(trans-(l)-1,2-diaminocyclohexan)platin(II)] nitrat,
Hydroxo(äthylharnstoff-N,O)-bis [cis-(trans-(d)-1,2-diaminocyclohexan)platin(II)] nitrat,
Hydroxo(äthylharnstoff-N,O)-bis [cis-(cis-1,2-diaminocyclohexan)platin(II)] nitrat,
Bis(äthylharnstoff-N,O)-bis [cis-trans(l)-1, 2-diaminocyclohexan)platin (II)] nitrat,
Bis(äthylharnstoff-N,O)-bis [cis-(trans(d)-1,2-diaminocyclohexan)platin(II)] nitrat oder
Hydroxo(propylharnstoff-N,O)-bis(cis-(trans-(d)-1,2-diaminocyclohexan)platin(II)] nitrat
hergestellt wird.

7. Verwendung einer Verbindung der Formel (I) für die Herstellung eines Heilmittels zum Behandeln eines an einem Tumor leidenden Tieres, worin die Formel so wie in Anspruch 1 definiert ist.